(19) Europäisches Patentamt
European Patent Office
Office européen des brevets

(11) Publication number: **0 003 857**
**B1**

(12) # EUROPEAN PATENT SPECIFICATION

(45) Date of publication of patent specification: 10.03.82

(51) Int. Cl.³: C 07 C 121/40, B 01 J 31/20

(21) Application number: 79200073.9

(22) Date of filing: 09.02.79

(54) **Preparation of 2-cyanohexanoic acid derivatives.**

(30) Priority: 20.02.78 GB 672278
24.05.78 GB 2191178

(43) Date of publication of application:
05.09.79 Bulletin 79/18

(45) Publication of the grant of the patent:
10.03.82 Bulletin 82/10

(84) Designated Contracting States:
BE CH DE FR GB IT NL

(56) References cited:
FR - A - 1 409 516
FR - A - 2 357 534
GB - A - 1 520 024
GB - A - 1 526 422
LU - A - 73 349

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Sheldon, Roger Arthur
Badhuisweg 3
NL-1031 CM Amsterdam (NL)
Inventor: Anderson, Martin
92 Clare Road
Whitstable, Kent (GB)
Inventor: Verbrugge, Herman
Badhuisweg 3
NL-1031 CM Amsterdam (NL)
Inventor: Syrier, Johannes Leopold Marie
Badhuisweg 3
NL-1031 CM Amsterdam (NL)

(74) Representative: Rogers, Roy Charles et al,
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 003 857 B1

Courier Press, Leamington Spa, England.

# Preparation of 2-cyanohexanoic acid derivatives

This invention relates to a process for the preparation of 2-cyanohexanoic acid derivatives which are useful intermediates in the manufacture of insecticidally-active compounds.

The 2-cyanohexanoic acid derivatives may be cyclized by a base to form substituted cyclopropane compounds and these compounds may be used in the manufacture of esters of cyclopropane carboxylic acids, a group of insecticidally-active compounds known as "synthetic pyrethroids".

In the Applicant's pending application, Netherlands patent application 7707416 there are disclosed 2-cyano-4,6,6,6-tetrahalohexanoic acid derivatives as novel compounds as well as a method for their preparation. This method involves the reaction of a 2-cyano-4-pentenoic acid derivative with a tetrahalomethane in the presence of a catalyst and a solvent capable of dissolving the reactant and the catalyst. The catalysts disclosed are ferric, cupric or ruthenium chloride, naphthenate, stearate or acetylacetonate and they are used in the presence of (a), a solubilizer for the catalyst such as an alkylammonium halide and (b), a reducing agent, such as benzoin. These additional compounds in the reaction mixture cause difficulties in the working-up procedure and, moreover, the presence of benzoin causes certain effluent disposal problems. As the reaction usually proceeds at a temperature of about 100°C and as the tetrahalomethane is a gas at this temperature the reaction has to be conducted at superatmospheric pressure thereby involving the use of equipment that will withstand high pressure.

The Applicant has found a modification of this earlier process which permits it to be carried out at atmospheric pressure and also avoids the need to use additional compounds, such as alkylammonium halides and benzoin.

Accordingly, the present invention provides a process for the preparation of 2-cyanohexanoic acid derivatives having the general formula:

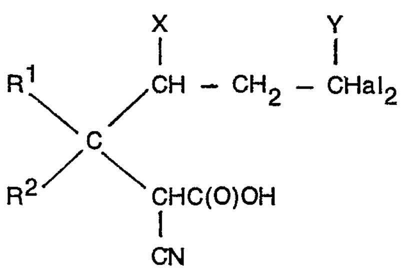
(I)

wherein X and Y each represent a chlorine or bromine atom, each Hal represents a fluorine, chlorine or bromine atom and $R^1$ and $R^2$ each independently represent an alkyl group of one to four carbon atoms, which comprises reacting a pentanoic acid compound of the general formula:

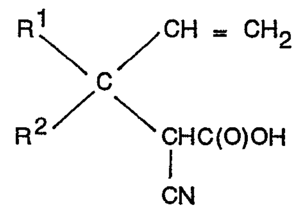
(II)

wherein $R^1$ and $R^2$ have the same meaning as in the general formula I with a tetrahalomethane of the general formula:

$$CHal_2XY \qquad (III)$$

wherein Hal, X and Y have the same meaning as in the general formula I, in the presence of a catalyst and a solvent for the reactants, characterized in that the catalyst is a transition metal in a divided state belonging to Groups VIB, VIIB or VIII of the Periodic Table of the Elements or a carbonyl of such a transition metal.

The alkyl groups represented by $R^1$ and $R^2$ are preferably both methyl groups and thus the preferred compound of general formula II is 2-cyano-3,3-dimethyl-4-pentenoic acid.

Examples of the tetrahalomethane of the general formula III are carbon tetrachloride, carbon tetrabromide, bromotrichloromethane, dibromodichloromethane, trichlorofluoromethane, and dibromodifluoromethane. Carbon tetrachloride is the preferred compound of the general formula III.

The reaction product from the addition of carbon tetrachloride to 2-cyano-3,3-dimethyl-4-pentenoic acid is 4,6,6,6-tetrachloro-2-cyano-3,3-dimethylhexanoic acid, i.e. the compound of general formula I, wherein $R^1 = R^2 =$ methyl and X = Y = Hal = chlorine.

The solvent for the reactants is preferably the tetrahalomethane of the general formula III, and under these circumstances excess tetrahalomethane over the amount required as reactant will have to be employed. Other examples of useful solvents are ketones and ethers, for example di-isobutyl ketone and tetrahydrofuran; tetrahydrothiophene-1,1-dioxide and dimethyl sulphoxide.

Examples of suitable transition metal catalysts which can be used in the process according to the invention in a divided state or in the form of their carbonyls are chromium, molybdenum, tungsten, manganese, rhenium, iron, ruthenium, osmium, cobalt, rhodium, iridium or nickel. Particular examples of carbonyls are $Cr(CO)_6$, $Mo(CO)_6$, $W(CO)_6$,

$[Mn(CO)_5]_2$, $[Re(CO)_5]_2$, $Fe(CO)_5$, $Fe_2(CO)_9$, $Ru(CO)_5$, $Os(CO)_5$, $[Co(CO)_4]_2$, $[Co(CO)_3]_4$, $[Rh(CO)_4]_2$, $[Ir(CO)_4]_2$ and $Ni(CO)_4$. Very good results have been obtained with transition metals of Group VIII of the Periodic Table, especially iron in a divided state or an iron carbonyl. When a transition metal is employed it is preferably in a finely divided or particulate state, e.g., in the form of a powder, filings, balls or shavings. Such metals are generally less toxic, less expensive and safer to use than their corresponding carbonyls and are therefore preferred.

When carbonyls are employed as catalysts in the process according to the invention they may, if desired, be prepared *in situ* for example by introducing iron and carbon monoxide into the reaction vessel.

The ratio of the transition metal or its carbonyl to the pentenoic acid compound of the general formula II is not critical and may vary within a wide range. Preferably, the ratio of the number of gram atoms of transition metal to the number of moles of compound of general formula II is in the range of from 0.0001:1 to 1:1.

It has been found that improved yields are very often obtained when a sequestering agent capable of binding ions of transition metals is added to the reaction mixture and it is believed that its role is a suppressant for minor undesired side reactions. The presence of this sequestering agent usually increases the selectivity to the 2-cyanohexanoic acid derivatives of the general formula I to 100% or almost 100%. The selectivity to the derivatives of the general formula I expressed in a percentage, is defined as:

$$\frac{a}{b} \times 100,$$

wherein "a" is the amount of the pentenoic acid compound of formula II converted into the compound of formula I and "b" is the amount of converted compound of formula II. Alkanenitriles are very suitable sequestering agents, particularly those with fewer than six carbon atoms per molecule; very good results have been obtained with acetonitrile. Other examples of sequestering agents are alkanamides, N-alkyl-substituted alkanamides and N,N-dialkyl-substituted alkanamides, particularly those with fewer than six carbon atoms per molecule, for example N-methylformamide and N,N-dimethylformamide.

The molar ratio of sequestering agent to the transition metal of Group VIB, VIIB or VIII or the carbonyl thereof is not critical and may vary within a wide range. A suitable range for this molar ratio is from 0.1:1 to 500:1, particularly of from 1:1 to 100:1.

The process according to the present invention may be carried out at atmospheric pressure and at a temperature in the range of, for example, 50 to 100°C. Thus, when carbon tetrachloride is the tetrahalomethane of the general formula III, the process may be carried out under reflux (for example 77°C) and at atmospheric pressure.

According to a preferred feature of the present invention, the transition metal or its carbonyl is gradually added to the reaction mixture comprising the compounds of the general formulae II and III and the solvent for the reactions during the course of the reaction. If desired, this mixture may already contain part of the transition metal or its carbonyl. The period of addition may vary, between, say, two and ten hours. A sequestering agent, when used, may be gradually added during the reaction, in admixture with the transition metal or its carbonyl, and/or be present at the start of the reaction.

The reaction mixture obtained may be filtered as necessary, washed with an aqueous solution of a mineral acid and the solvent evaporated from the washed liquid to yield a residue containing the compound of the general formula I.

The Periodic Table referred to in this specification is the version shown on pages 448 and 449 of "Handbook of Chemistry and Physics", 4th Edition published by The Chemical Rubber Publishing Co.,

The following Examples further illustrate the invention with reference to the preparation of 4,6,6,6-tetrachloro-2-cyano-3,3-dimethylhexanoic acid from 2-cyano-3,3-dimethyl-4-pentenoic acid. These acids are referred to in the Examples as "product acid" and "starting acid", respectively. The reactions were carried out in a glass flask equipped with a magnetic stirrer.

Example 1

A glass flask, equipped with a magnetic stirrer, was charged with starting acid (2.5 mols), carbon tetrachloride (3.725 mols) and acetonitrile (5 mols) and heated to reflux temperature (70°C). Iron powder having an average diameter of 150 $\mu$ (0.3 gram atoms) was gradually added to the refluxing reaction mixture over a period of five hours after which the mixture was allowed to react for a further period of two hours. The conversion of the starting acid was 97%m and the selectivity to the product acid was 85%m.

The reaction mixture was then filtered, washed with 2N aqueous sulphuric acid and then washed with aqueous sodium bicarbonate. The aqueous phase obtained was extracted with methylisobutyl ketone and the resultant aqueous phase acidified with 2N aqueous sulphuric acid. The precipitated product acid and unreacted starting acid were then isolated by filtration. The yield of product acid was 80%m.

Example 2

The flask was charged with starting acid (32.6 m.mol.), iron pentacarbonyl (0.91 m.mol.), carbon tetrachloride (117 m.mol.) and acetonitrile (19.2 m.mol.). The solution thus formed was heated under reflux (77°C) whilst a solution of iron pentacarbonyl (5.9 m.Mol.) in acetonitrile (134 m.mol) was gradually added over a period of six hours. At the end of this period the conversion of the starting acid was 98% and the selectivity to the product acid 100%.

Subsequently, the contents of the flask were cooled to 20°C and washed with 2N aqueous sulphuric acid (50 ml). The washed liquid was dried over anhydrous sodium sulphate and the solvent was evaporated from the dried liquid at 19 m bar to give a residue in which the product acid was present in a yield of 88%.

Example 3

The flask was charged with starting acid (32.6 m.mol.), iron pentacarbonyl (0.91 m.mol.), carbon tetrachloride (117 m.mol.) and acetonitrile (1.95 m.mol.)., The solution thus formed was heated whilst a solution of iron pentacarbonyl (5.9 m.mol.) in acetonitrile (12.6 m.mol.) was gradually added over a period of six hours. At the end of this period the conversion of the starting acid was 98% and the selectivity to the product acid 100%.

Example 4

The flask was charged with starting acid (32.6 m.mol.), iron pentacarbonyl (3.3 m.mol.) and carbon tetrachloride (117 m.mol.). The solution thus formed was heated for a period of four hours. At the end of this period the conversion of the starting acid was 53%. The selectivity to the product acid was 66%. The byproducts mainly resulted from decarboxylation of the starting and the product acid and the balance from dehydrohalogenation of the product acid.

**Claims**

1. A process for the preparation of 2-cyanohexanoic acid derivatives having the general formula:

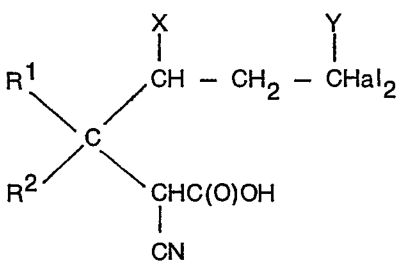

(I)

wherein X and Y each represent a chlorine or bromine atom, each Hal represents a fluorine, chlorine or bromine atom and $R^1$ and $R^2$ each independently represent an alkyl group of one to four carbon atoms, which comprises reacting a pentenoic acid compound of the general formula:

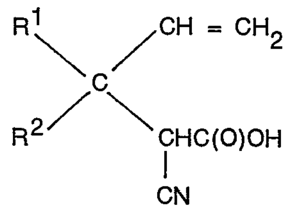

(II)

wherein $R^1$ and $R^2$ have the same meaning as in the general formula I with a tetrahalomethane of the general formula:

$$CHal_2XY \qquad (III)$$

wherein Hal, X and Y have the same meaning as in the general formula I, in the presence of a catalyst and a solvent for the reactants, characterized in that the catalyst is a transition metal in a divided state belonging to Groups VIB, VIIB or VIII of the Periodic Table of the Elements or a carbonyl of such a transition metal.

2. A process according to claim 1, characterized in that the catalyst is chromium, molybdenum, tungsten, manganese, rhenium, iron, ruthenium, osmium, cobalt, rhodium, iridium or nickel in a divided state, or a carbonyl thereof.

3. A process according to claim 1 or 2, characterized in that the catalyst is iron in a divided state or an iron carbonyl.

4. A process according to any one of the preceding claims, characterized in that the solvent is the tetrahalomethane of formula III used in excess.

5. A process according to any one of the preceding claims, characterized in that the process is carried out in the presence of a sequestering agent capable of binding transition metal ions.

6. A process according to claim 5, wherein the sequestering agent is acetonitrile.

7. A process according to any one of the preceding claims, characterized in that the process is carried out at a temperature in the range 50 to 100°C.

8. A process according to any one of the preceding claims, characterized in that the transition metal or its carbonyl is gradually added to the reaction mixture during the course of the reaction.

9. A process according to claim 8, characterized in that the transition metal or its carbonyl is added to the reaction mixture over a period of 2 to 10 hours.

## Revendications

1. Procédé pour la préparation de dérivés d'acide 2-cyanohexanoïque ayant la formule générale:

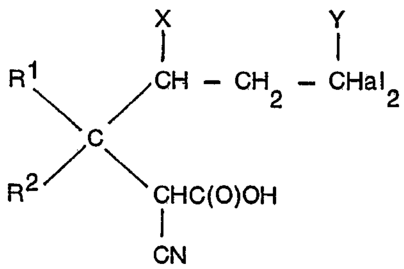

(I)

dans laquelle X et Y représentent chacun un atome de chlore ou de brome, chaque Hal représente un atome de fluor, de chlore ou de brome et $R^1$ et $R^2$ représentent chacun indépendamment un groupe alcoyle ayant de 1 à 4 atomes de carbone, selon lequel on fait réagir un dérivé d'acide penténoïque de la formula générale:

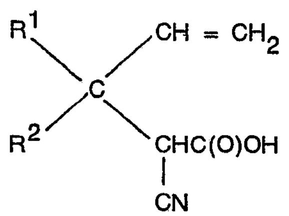

(II)

dans laquelle $R^1$ et $R^2$ ont la même signification que dans la formule générale I, avec un tétrahalométhane de la formule générale:

$$CHal_2XY \qquad (III)$$

dans laquelle Hal, X et Y, ont la même signification que dans la formule générale I, en présence d'un catalyseur et d'un solvent pour les corps en réaction, caractérisé en ce que le catalyseur est un métal de transition dans un état divisé appartenant aux groupes VIB, VIIB ou VIII du tableau périodique des éléments ou un carbonyle d'un tel métal de trasition.

2. Procédé selon la revendication 1, caractérise en ce que le catalyseur est du chrome, du molybdène, du tungstène, du manganèse, du rhénium du fer, du ruthénium, de l'osmium, du cobalt, du rhodium, de l'iridium ou du nickel dans un état divisé ou un carbonyle de ces métaux.

3. Procédé selon l'une quelconque des revendications 1 et 2, caractérisé en ce que le procédé est du fer dans un état divisé ou un fer-carbonyle.

4. Procédé selon l'une quelconque des revendications précédentes, caractérisé en ce que le solvant est le tétrahalométhane de formule III utilisé en excès.

5. Procédé selon l'une quelconque des revendications précédentes, caractérisé en ce que le procédé est mis en oeuvre en présence d'un agent séquestrant capable de fixer les ions de métaux de transition.

6. Procédé selon la revendication 5, caractérise en ce que l'agent séquestrant est l'acétonitrile.

7. Procédé selon l'une quelconque des revendications précédentes, caractérisé en ce que le procédé est mis en oeuvre à une température comprise entre 50 et 100°C.

8. Procédé selon l'une quelconque des revendications précédentes, caractérisé en ce que le métal de transition ou son carbonyle est ajouté progressivement au mélange réactionnel durant le cours de la réaction.

9. Procédé selon la revendication 8, caractérisé en ce que le mélange de transition ou son carbonyle est ajouté un mélange réactionnel en une période de 2 à 10 heures.

## Patentansprüche

1. Verfahren zur Herstellung von Derivaten der 2-Cyano-hexansäure der allgemeinen Formel:

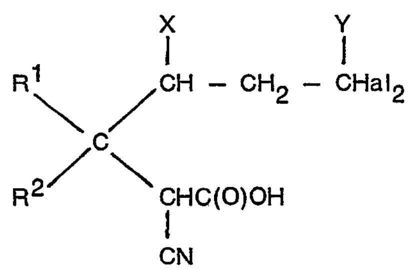

(I)

worin X und Y je ein Chlor- oder Bromatom, jedes Hal ein Fluor-, Chlor- oder Bromatom und $R^1$ und $R^2$ unabhängig von einander je eine Alkylgruppe mit 1 bis 4 Kohlenstoffatomen bedeuten, wobei man eine Pentensäure der allgemeinen Formel:

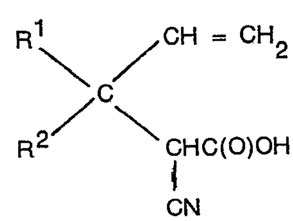

(II)

worin $R^1$ und $R^2$ dieselbe Bedeutung haben wie in der allgemeinen Formel I, umsetzt mit einem Tetrahalogenmethan der allgemeinen Formel:

$$CHal_2XY \qquad (III)$$

worin Hal, X und Y dieselbe Bedeutung haben wie in der allgemeinen Formel I, in Gegenwart eines Katalysators und eines Lösungsmittels für

die Reaktionsteilnehmer, dadurch gekennzeichnet, das man als Katalysator eine in feinverteiltem Zustand befindliches Übergangsmetall der Gruppen VIB, VIIB oder VIII des Periodensystems oder ein Carbonyl eines solchen Übergangsmetalls verwendet.

2. Verfahren nach Anspruch 1, dadurch gekennzeichnet, dass man als Katalysator Chrom, Molybdän, Wolfram, Mangan, Rhenium, Eisen, Ruthenium, Osmium, Kobalt, Rhodium, Iridium oder Nickel in feinverteiltem Zustand oder eines ihrer Carbonyle verwendet.

3. Verfahren nach Anspruch 1 oder 2, dadurch gekennzeichnet, dass man als Katalysator feinverteiltes Eisen oder ein Eisencarbonyl verwendet.

4. Verfahren nach einem der vorangehenden Ansprüche, dadurch gekennzeichnet, dass man als Lösungsmittel das Tetrahalogenmethan nach Formel III im Überschuss verwendet.

5. Verfahren nach einem der vorangehenden Ansprüche, dadurch gekennzeichnet, dass man es in Anwesenheit eines zum Binden von Übergangsmetallionen fähigen Abfangmittels durchführt.

6. Verfahren nach Anspruch 5, dadurch gekennzeichnet, dass man als Abfangmittel Acetonitril verwendet.

7. Verfahren nach einem der vorangehenden Ansprüche, dadurch gekennzeichnet, dass man die Umsetzung bei einer Temperatur von 50 bis 100°C durchführt.

8. Verfahren nach einem der vorangehenden Ansprüche, dadurch gekennzeichnet, dass man das Übergangsmetall bzw. dessen Carbonyl während des Reaktionsverlaufes allmählich dem Reaktionsgemisch zufügt.

9. Verfahren nach Anspruch 8, dadurch gekennzeichnet, dass man das Ubergangsmetall bzw. dessen Carbonyl dem Reaktionsgemisch innerhalb von 2 bis 10 Stunden zufügt.